# EUROPEAN PATENT SPECIFICATION

(11) **EP 0 801 133 B1**
(45) Date of publication and mention of the grant of the patent: **08.09.2004**
(21) Application number: 97105347.5
(22) Date of filing: 29.03.1997
(51) Int. Cl.: C12N 15/52, C12N 9/88, C12P 7/24, C12P 7/40, C12P 7/04, C11B 9/00

(54) **Hydroperoxide lyases**
Hydroxyperoxide Lyasen
Lyases de hydroxy-peroxyde

(30) Priority: 15.04.1996 EP 96105856; 25.09.1996 EP 96115335
(43) Date of publication of application: 15.10.1997
(73) Proprietor: Givaudan SA, 1214 Vernier-Genève (CH)
(72) Inventor: Häusler, Alex, 8603 Schwerzenbach (CH); Lerch, Konrad, 8118 Pfaffhausen (CH); Muheim, Andreas, 8002 Zürich (CH); Silke, Natasha, 8047 Zürich (CH)
(74) Representative: McStea, John Anthony

(56) References cited:
- EP-A- 0 481 147
- WO-A-95/26413
- VICK, BRADY A.: "Temporal and organ-specific expression of enzymes of fatty acid hydroperoxide metabolism in developing sunflower seedlings" PLANT LIPID METAB., [PAP. INT. MEET. PLANT LIPIDS], 11TH (1995), MEETING DATE 1994, 280-2. EDITOR(S): KADER, JEAN-CLAUDE;MAZLIAK, PAUL. PUBLISHER: KLUWER, DORDRECHT, NETH. ,1995, XP002120137
- HATANAKA A ET AL: "EXPRESSION OF LIPOXYGENASE AND HYDROPEROXIDE LYASE ACTIVITIES IN TOMATO FRUITS." Z NATURFORSCH SECT C BIOSCI, (1992) 47 (5-6), 369-374. , XP002120138

## Description

The present invention relates to hydroperoxide lyases (hereinafter also referred to as HPO lyase proteins or proteins with HPO lyase activity), their microbial production via recombinant DNA technology, and their use for the production of aliphatic aldehydes and alcohols, flavor molecules known "as green notes".

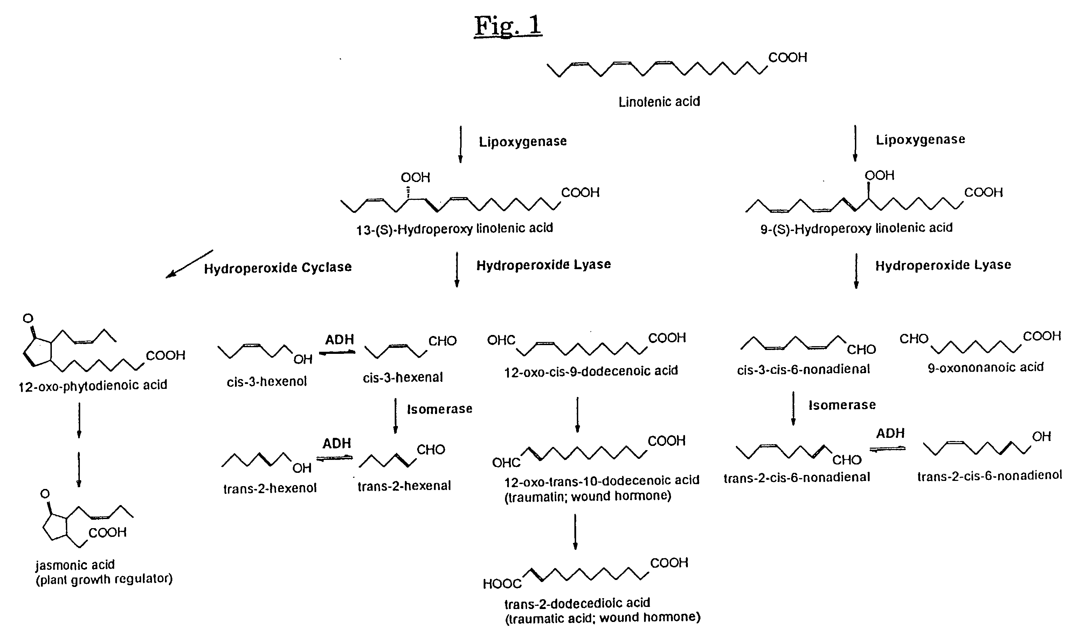

"Green notes" are volatile flavor and fragrance molecules present in a wide variety of plant leaves, vegetables and fruits characterized in organoleptic terms as fresh "green" and grassy. These compounds are produced by the plant from the degradation of unsaturated fatty acids (linoleic and linolenic acid). In Fig. 1 the formation of a variety of linolenic acid degradation products is summarized.

Degradation of polyunsaturated fatty acids starts by the oxygenation at cis-cis double bonds of polyunsaturated fatty acids. This reaction is catalyzed by lipoxygenase (EC 1.13.11.12)-enzymes which are present in plants, animals and microorganisms. The oxygenated products, fatty acid hydroperoxides, are precursors for many important hormones (e.g. prostaglandins, lipoxins, jasmonic acid, traumatic acid) and flavor/fragrance molecules (e.g., cis-3-hexenol, 1-octen-3-ol). In plants, cleavage of the hydroperoxides occurs through the action of specific hydroperoxide lyases.

Commercial production of natural "green note" compounds is currently achieved by fractional distillation of essential oils such as mint oil or by the combined action of lipoxygenase and hydroperoxide lyase on unsaturated fatty acids using plant material from different sources.

WO-A-9 526 413 discloses a process for providing green note compounds using plant tissue. Other documents describe the level of expression of lipoxygenase and hydroperoxide lyase in different sunflower organs [Vick, Plant Lipid Metabolism, 280-282 (1995)] and in tomato fruits [Hatanaka et al., Biosciences 47, 369-374 (1992)].

However, these processes have the drawbacks that they provide low yields and/or depend on specific plant materials.

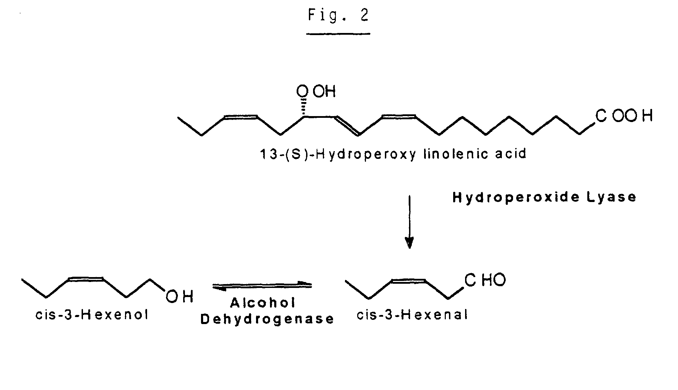

It has now been found that high reproducible yields of "green note" compounds (e.g., cis-3-hexenol) can be obtained independent of plant materials and in the absence of unwanted side reaction (e.g. isomerase activity) by transfer of the gene coding for HPO lyase from plant into host cells, subsequent expression of the gene, addition of linolenic acid hydroperoxide as substrate, and reduction of cleaved substrate by aldehyde dehydrogenase. In Fig. 2 the formation of cis-3-hexenol from 13-(S)-hydroperoxy linolenic acid by recombinant HPO lyase is summarized.

Thus, in a first aspect of this invention, there are provided isolated DNA sequences encoding proteins with HPO lyase activity, wherein the DNA sequences of this invention are defined to include the nucleotide sequence SEQ ID No:1 or a fragment thereof or any DNA sequence which is substantially homologous to the nucleotide sequence SEQ ID No:1 or a fragment thereof.

As used hereinbefore the term "substantially homologous", means that a particular subject sequence, for example, a mutant sequence, varies from a reference sequence by one or more substitutions, deletions, or additions, the net effect of which does not result in an adverse functional dissimilarity between the reference and subject sequences. For purposes of the present invention, DNA sequences having greater than 95 percent homology, encoding equivalent biological properties, and showing equivalent expression characteristics are considered substantially homologous. For purposes of determining homology, truncation of the DNA sequence should be disregarded. Sequences having lesser degrees of homology, encoding comparable bioactivity, and showing equivalent expression characteristics, e.g., fragments of the nucleotide sequence SEQ ID No:1 are considered substantial equivalents. Generally, homologous DNA sequences can be identified by cross-hybridization under standard hybridization conditions of moderate stringency.

There are also provided vectors and expression vectors containing the DNA sequences of the present invention, hosts containing such vectors for the production of proteins with HPO lyase activity, and processes for the production of such DNA sequences, recombinant vectors and host cells.

There are further provided recombinant proteins with HPO lyase activity. Specifically a protein with HPO lyase activity is defined to include the amino acid sequence SEQ ID No:2 or any protein or polypeptide having an amino acid sequence which is substantially homologous to the amino acid sequence SEQ ID No:2 and further having the following biological activity: When the protein or polypeptide is incubated under suitable conditions and a suitable amount of substrate such as 13-(S)-linolenic acid hydroperoxide is added, the formation of cis-3-hexenal is observed.

As used hereinbefore the term "substantially homologous" means that a particular subject sequence, for example, a mutant sequence, varies from a reference sequence by one or more substitutions, deletions, or additions, the net effect of which does not result in an adverse functional dissimilarity between the reference and subject sequences. For purposes of the present invention, sequences having greater than 95 percent homology, equivalent biological activity and equivalent expression characteristics are considered substantially homologous. For purposes of determining homology, truncation of the sequence should be disregarded. Sequences having lesser degrees of homology, comparable bioactivity, and equivalent expression characteristics, e.g., fragments of the amino acid sequence SEQ ID No:2 are considered substantial equivalents.

As used herein the term recombinant proteins with HPO lyase activity includes proteins modified deliberately, as for example, by addition of specific sequences that preferably bind to an affinity carrier material. Examples of such sequences are sequences containing at least two adjacent histidine residues (see in this respect European Patent No. 282 042). Such sequences bind selectively to nitrilotriacetic acid nickel chelate resins (Hochuli and Döbeli, Biol. Chem. Hoope-Seyler 368, 748 (1987); European Patent No. 253 303). Proteins with HPO lyase activity which contain such a specific sequence can, therefore, be separated selectively from the remaining polypeptides. The specific sequence can be linked either to the C-terminus or the N-terminus of the proteins with HPO lyase activity.

Methods for the expression, isolation and purification of the proteins with HPO lyase activity are also provided.

The following steps outline the methods for recombinantly expressing the proteins with HPO lyase activity.

### 1) Cloning of DNA sequences encoding proteins with HPO lyase activity

DNA sequences encoding proteins with HPO lyase activity can be cloned using a variety of techniques. Using the methods described in this application cDNAs encoding proteins with HPO lyase activity or fragments thereof can be produced. These cDNAs can be isolated and amplified by PCR technique using oligodeoxynucleotide DNA primers by conventional techniques.

The cDNA (SEQ ID No:1) encoding the amino acid sequence SEQ ID No:2 is obtained using the DNA primers described in the examples. By using conventional technique, this cDNA has been isolated from a lambda phage cDNA library made from RNA derived from banana (Musa sp.) leaves.

The cDNA may be obtained not only from cDNA libraries, but by other conventional techniques, e.g., by cloning genomic DNA, or fragments thereof, purified from the desired cells. These procedures are described by Sambrook et al., in "DNA Cloning: A Practical Approach", Vol. I and II, D.N. Glover, ed., 1985, MRL Press, Ltd., Oxford, U.K.; Benton and Davis, Science 196, 180-182 (1977); and Grunstein and Hogness, Proc. Nat. Acad. Sci. 72, 3961-3965 (1975).

To obtain the cDNA encoding the proteins with HPO lyase activity cDNA libraries are screened by conventional DNA hybridization techniques by the methods of Benton and Davis, supra, or Grunstein and Hogness, supra, using radioactive HPO lyase gene fragments. Clones which hybridize to the radioactive gene fragments are analyzed, e.g., by restriction endonuclease cleavage or agarose gel electrophoresis. After isolating several positive clones the positive insert of one clone is subcloned, e.g., into phagemids, and sequenced by conventional techniques.

Clones derived from genomic DNA may contain regulatory and intron DNA regions in addition to coding regions; clones derived from cDNA will not contain intron sequences. In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Whatever the source, the DNA sequence encoding proteins with HPO lyase activity may be moleculary cloned into a suitable vector for propagation of the DNA by methods known in the art. Any commercially available vector may be used. For example, the DNA may be inserted into a pBluescript SK-vector. Appropriate vectors for use with bacterial hosts are described by Pouwels et al., in "Cloning Vectors: A Laboratory Manual", 1985, Elsevier, N.Y. As a representative but nonlimiting example, useful cloning vectors for bacterial use can comprise a selectable marker and a bacterial origin of replication derived from commercially available plasmids which are in turn derived from the well known cloning vector pBR322 (ATCC 37017). Such commercial vectors include, for example, pKK223-3 (Pharmacia Fine Chemicals, Uppsala, Sweden) and pGEM1 (Promega Biotec, Madison, Wisc., USA).

The DNA sequences encoding proteins with HPO activity inserted in these commercially available vectors can be verified by methods known in the art, e.g., by standard nucleotide sequencing techniques.

DNA sequences that code for proteins with HPO activity from plants other than banana may be used herein. Accordingly, while specific DNA has been cloned and sequenced in relation to the DNA sequence in banana leaves, any plant cell potentially can be used as the nucleic acid source of the protein with HPO activity.

### 2) Production of proteins with HPO lyase activity

Cloned DNA sequences that code for proteins with HPO lyase activity can be expressed in hosts to enable the production of these proteins with greater efficiency. Techniques for these genetic manipulations are specific for the different available hosts and are known in the art.

For expression of proteins with HPO lyase activity in hosts, in principle, all vectors which replicate and express DNA sequences encoding the proteins with HPO lyase activity in the chosen host are suitable. Expression vectors suitable for use in prokaryotic host cells are mentioned, for example, in the textbook "Molecular Cloning - A Laboratory Manual", Cold Spring Harbor Laboratory (1982), of Maniatis et al. Examples of other vectors are plasmids of the pDS family [Bujard et al., Methods in Enzymology, eds. Wu and Grossmann, Academic Press, Inc., Vol. 155, 416-433 (1987)].

Such prokaryotic expression vectors which contain the DNA sequences coding for the proteins with HPO lyase activity operatively linked with an expression control sequence can be incorporated using conventional methods into any suitable prokaryotic host cell. The selection of a suitable prokaryotic host cell is determined by different factors which are well-known in the art. Thus, for example, compatibility with the chosen vector, toxicity of the expression product, expression characteristics, necessary biological safety precautions and costs play a role and a compromise between all of these factors must be found.

Suitable prokaryotic host organisms include gram-negative and gram-positive bacteria, for example E. coli and B. subtilis strains. Examples of prokaryotic host organisms are E. coli strain M15 (described as strain OZ 291 by Villarejo et al. in J. Bacteriol. 120, 466-474 [1974] and E. coli W3110 [ATCC No. 27325]). In addition to the aforementioned E. coli strains, however, other generally accessible E. coli strains such as E. coli 294 (ATCC No. 31446) and E. coli RR1 (ATCC No. 31343) can also be used.

In a preferred embodiment of the present invention yeast is used as the host organism. Expression vectors suitable for use in yeast cells are described in "Guide to yeast genetics and molecular biology", Guthrie and Fink, eds., Methods in Enzymology, Academic Press, Inc., Vol. 194 (1991) and "Gene expression technology", Goeddel, ed., Methods in Enzymology, Academic Press, Inc., Vol. 185 (1991). The preferred yeast vector of the present invention is the plasmid pYX233 (R&D systems, Abingdon, UK). Examples of suitable yeast cells are Saccharomyces cerevisiae, Pichia pastoris, Hansenula polymorpha, Schizosaccharomyces pombe cells. An overview on various yeast expression systems is given by Romanos et al., Yeast, Vol. 8, 423-488 (1992). Especially preferred yeast cells of the present invention are S. cerevisiae DBY746 [ATCC 44773].

The transformation with the yeast expression vectors is carried out as described by Klebe et al., Gene, Vol. 25, 333-341 (1983).

The manner in which the expression of the proteins with HPO lyase activity is carried out depends on the chosen expression vector host cell system.

Usually, the prokaryotic host cells which contain a desired expression vector are grown under conditions which are optimal for the growth of the prokaryotic host cells. At the end of the exponential growth, when the increase in cell number per unit time decreases, the expression of the desired protein with HPO lyase activity is induced, i.e., the DNA coding for the desired protein with HPO lyase activity is transcribed and the transcribed mRNA is translated. The induction can be carried out by adding an inducer or a derepressor to the growth medium or by altering a physical parameter, e.g., a change in temperature. For example, the expression can be controlled by the lac repressor.

By adding isopropyl-β-D-thiogalactopyranoside (IPTG), the expression control sequence is derepressed and the synthesis of the desired protein is thereby induced.

The yeast host cells which contain a desired expression vector are grown under conditions which are optimal for the growth of the yeast host cells. A typical expression vector contains the promoter element, which mediates the transcription of mRNA, the protein coding sequence, a ribosomal binding site for effective translation. Additional elements may include terminator, signal, and upstream activating sequences.

The yeast cells are grown as described by Sherman in "Guide to yeast genetics and molecular biology", Guthrie and Fink, eds., Methods in Enzymology, Academic Press, Inc., Vol. 194, 3-21 (1991).

The baculovirus-insect cell vector system can also be used for the production of the proteins with HPO lyase activity of the present invention (for review see Luclow and Summers, Bio Technology 6, 47-55 [1988]). The proteins with HPO lyase activity produced in insect cells infected with recombinant baculovirus can undergo post-translational processing including N-glycosylation (Smith et al., Proc. Nat. Scad. Sci. USA 82, 8404-8408) and O-glycosylation (Thomsen et al., 12. International Herpesvirus Workshop, University of Philadelphia, Pennsylvania).

Plants can also be used as hosts for the recombinant production of proteins with HPO lyase activity. Transfer of the gene coding for the protein with HPO lyase activity may be achieved by a variety of methods (for review see Potrykus and Spangenberg, eds., Gene transfer to plants. A laboratory manual, Springer Verlag, Heidelberg, Germany (1995)), whereby the HPO lyase gene is integrated into the chromosome of the host plant. Homologous expression - overexpression - of the protein with HPO lyase activity can be achieved, for example, by transforming a banana (Musa sp.) plant with the HPO lyase gene isolated from a banana gene library. A transformation protocoll of banana plants can be found in Bio Technology 13 (5), 486-492 (1995) or Bio Technology 13 (5), 481-485 (1995). Other examples for plant hosts for the production of recombinant HPO lyase protein include, but are not limited to maize (Zea mays, Ishida et al., Nature Biotechnology 14, 745-750 (1996)), flax (Linum usitatissimum, Dong and Mchughen, Plant Sci. 88 (1), 61-71 (1993)) and soybean (Glycine max, Christou et al., Tibtech 8, 145-151 (1990)).

For the isolation of small amounts of proteins with HPO lyase activity expressed in prokaryotic host cells for analytical purposes, e.g., for polyacrylamide gel electrophoresis, the host cells can be disrupted by treatment with a detergent, e.g., sodium dodecyl sulphate (SDS). Larger quantities of the HPO lyase protein can be obtained by mechanical [Charm et al., Meth. Enzymol. 22, 476-556 (1971)], enzymatic (lysozyme treatment) or chemical (detergent treatment, urea or guanidinium hydrochloride treatment, etc.) treatments followed by use of known methods, e.g., by centrifugation at different gravities, precipitation with ammonium sulphate, dialysis (at normal pressure or at reduced pressure), preparative isoelectric focusing, preparative gel electrophoresis or by various chromatographic methods such as gel filtration, high performance liquid chromatography (HPLC), ion exchange chromatography, reverse phase chromatography and affinity chromatography (e.g., on Sepharose® Blue CL-6B).

For the isolation of small amounts of proteins with HPO lyase activity expressed in yeast host cells for analytical purposes, e.g., for polyacrylamide gel electrophoresis, the host-cells can be disrupted by the use of glass beads as described by Orlean et al. in "Guide to yeast genetics and molecular biology", Guthrie and Fink, eds., Methods in Enzymology, Academic Press, Inc., Vol. 194, 682-697 (1991). Larger quantities of the proteins with HPO lyase activity can be obtained by passing recombinant yeast cells through a Dyno-Mill apparatus filled with glass beads according to the instructions of the manufacturer (Willy Bachofen, Maschinenfabrik AG, Basel, Switzerland).

The proteins with HPO lyase activity expressed in the baculovirus-insect cell vector system can be isolated from the host cell medium using standard protein purification methods.

The proteins with HPO lyase activity can be used in the production of natural "green note" compounds by catalyzing the formation of aldehydes from fatty acid hydroperoxide.

Hence the present invention also provides a process for the production of natural "green note" compounds, which process comprises the steps of:
a) reacting fatty acid hydroperoxide with recombinant proteins with HPO lyase activity; and
b) reacting the resulting aliphatic aldehydes with isomerase and/or alcohol dehydrogenase.

The term "green note" compounds relates to leaf aliphatic aldehydes and leaf aliphatic alcohols, e.g., cis-3-hexenol and trans-2-hexenal. Examples of fatty acid hydroperoxides are given in Fig. 1.

In the process for the production of natural "green note" compounds the proteins with HPO lyase activity can be used in isolated form, or alternatively, in form of cell-free extracts obtained from host cells containing vectors for the production of protein with HPO lyase activity.

In a preferred specific embodiment of the present invention, the process for the production of natural "green note" compounds is employed to produce cis-3-hexenol. The specific process for producing cis-3-hexenol comprises the steps of:
a) reacting 13-(S)-hydroperoxide linolenic acid with recombinant proteins with HPO lyase activity; and
b) reducing the resulting cis-3-hexanal with alcohol dehydrogenase.

In performing the specific process for producing cis-3-hexenol, preferably the proteins with HPO lyase activity are obtained from Saccharomyces cerevisiae cells containing vectors for the production of said proteins and reduction of cis-3-hexenal to cis-3-hexenol is catalyzed be endogeneous aldehyde dehydrogenase. Fig. 2 summarizes schematically this specific process.

The green note compounds, e.g. cis-3-hexenol, prepared by the process of the present invention can be used as odorant and/or flavorant and worked into odorant and/or flavorant compositions in a manner known per se.

Having now generally described this invention, the same will become better understood by reference to the specific examples, which are included herein for purpose of illustration only and are not intended to be limiting unless otherwise specified, in connection with the following figures:
- Fig. 1: summarizes schematically the degradation of linolenic acid by the lipoxygenase pathway in plants
- Fig. 2: summarizes schematically the formation of cis-3-hexenol from 13-(S)-hydroperoxy linolenic acid by recombinant HPO lyase protein and alcohol dehydrogenase.

Linolenic acid-(13*S*)-hydroperoxide was produced as described by Iacazio et al. (J. Org. Chem. **55**, 1690-1691 [1990]) using Lipoxygenase-1 from Fluka (62340; Fluka, Buchs, Switzerland). Linolenic acid (62159; Fluka, Buchs, Switzerland) was used as precursor. Typically, a 60 - 70 mM aqueous solution of linolenic acid hydroperoxide was obtained under these conditions. This precursor can be stored for several month in 0.5 ml aliquots at -80°C.

Enzyme activity of banana HPO lyase protein was measured as follows: The reaction volume was 500 µl containing 20 mM sodium phosphate buffer, pH 6.8, 0.8 mM linolenic acid hydroperoxide and 50 µl of banana HPO lyase protein in aqueous buffer. The reaction was incubated for 10 min at room temperature and subsequently stopped by the addition of 200 µl of methyl-t-butylether containing an internal standard such as cis-3-hexenol. Activity of the lyase was determined as function of the amount of cis-3-hexenal produced during this standard reaction. Cis-3-hexenal was quantified by capillary gaschromatography as described by Olias et al., J. Agric. Food Chem. Vol. 41, 2368-2373 (1993).

### Example 1

### Purification of banana HPO lyase protein

About 5 kg of banana (*Musa sp*.; purchased from a local store) tissue was used for the isolation of the HPO lyase protein. Operations were carried out at 4°C. Aliquots of 560 g of banana tissue were homogenized in 1.1 l of ice cold buffer A (50 mM sodium phosphate buffer, pH 6.8, 2 mM dithiothreitol, 7 mM EDTA, 0.25 mM PMSF) and 36 g of PVP K30 from Fluka using a Warring blender. The homogenate was centrifuged at 10'000 x g for 20 min and the supernatant (crude extract) was filtered through 3 layers of Miracloth (Calbiochem). The resulting filtrate was centrifuged at 100'000 x g for 50 min and the pellet was homogenized and solubilized in 150 ml buffer B (20 mM Tris, pH 7.0, 0.1 % Triton X114) and subsequently clarified by centrifugation at 100'000 x g for 30 min. The solubilized HPO lyase protein fraction was applied to a column of DEAE-CL6B (2.6 cm i.d. x 20 cm; Pharmacia) that was equilibrated with buffer A. HPO lyase protein was eluted with a linear gradient of 0 - 0.5 M ammonium acetate in buffer B at 2 ml/min. Fractions were collected and screened for activity of HPO lyase. Active fractions were pooled and concentrated by ultrafiltration with an Amicon ultrafiltration unit containing a PM30 membrane (Amicon). The concentrate, about 8 ml, was applied in 2 ml aliquots per chromatography run to a gelfiltration column (Superose 6, 23 mm i.d. x 50 cm, attached to a FPLC apparatus, Pharmacia). The flow rate was 1.5 ml/min of buffer B and fractions were collected and assayed for activity of HPO lyase. Active fractions were pooled and applied to an anion exchange chromatography (Poros 20 HQ, 4.6 mm i.d. x 100 mm, Perceptive Biosystems). The chromatography was performed on a BioCAD-Sprint workstation (Perseptive Biosytems) with a flow rate of 5 ml/min. The HPO lyase was eluted with a linear gradient of buffer C (20 mM Tris, pH 7.0, 0.2 % Triton X100 reduced) to buffer C containing 0.5 M ammonium acetate. Fractions were collected and assayed for HPO lyase activity. Active fractions were pooled, the pH was set to approximately 7.5 by dilution with buffer D (20 mM Tris pH 8.0, 0.2 % Triton X100 reduced) and reapplied to the Poros anion exchange column equilibrated with buffer D. The HPO lyase activity was eluted with a gradient of 0 - 0.4 M ammonium acetate in buffer D. Fractions were collected, assayed for HPO lyase activity and aliquots of each were analysed by SDS-polyacrylamide gel electrophoresis (SDS-PAGE). SDS-PAGE was performed as described by Ausubel et al., eds. "Current Protocols in Molecular Biology", (1995) published by Current Protocols, USA, using a Minigel apparatus (Hoeffer SE280). The gel was stained using a silver stain Plus kit (Bio-Rad) according to the manufacturers instructions. The HPO lyase was detected as protein band of about 55 kDA size. The specific activity was 6000 µmol cis-3-hexenal produced/hr/mg of protein. The protein activity was purified to more than 9000-fold.

Fractions containing the activity maxima from all repetitive purification runs were pooled and concentrated by precipitation. For this, the pooled fractions were mixed with two volumes of ethanol and cooled to -20°C for 5 hrs. The mixture was centrifuged at 20'000 x g for 30 min and the resulting pellet was washed with 70 % ethanol and air-dried for 15 min. The pellet was resuspended in 160 µl Tricine sample buffer (Novex, San Diego, USA) and the sample subjected to SDS-Tricine-PAGE (10-20%) (Novex, San Diego, USA). The protein bands were blotted onto a PVDF membrane (Immobilon PSQ, Millipore) with transfer buffer (10 mM 3-(cyclohexylamino)-1-propanesulfonic acid, 10% methanol, pH 11.0) for 60 min at 400 mA in a Trans-Blot cell (BioRad Laboratories, Richmond, California) and stained with Ponceau S (0.1% Ponceau S in 10% acetic acid). The stained bands were cut out of the gel and digested in 100 mM Tris-HCl, pH 8.0, containing 1% reduced Triton X-100 (RTX), 10% acetonitrile and 1 µg Lys-C overnight at 37°C. The samples were loaded on a Vydac C8 (250 x 1 mm) reverse-phase column. Sequence analysis of the eluted peptides was performed on a ABI Procise Protein Sequencer.

Amino acid sequences were obtained from 4 individual peptides as shown below (amino acid residues in parentheses are only tentatively assigned)

### Example 2

### Cloning of DNA encoding HPO lyase protein

### A. Isolation of RNA from banana leaves

Banana (Musa sp.) leaves containing high hydroperoxide lyase activity were frozen in liquid nitrogen and the tissue was powdered using mortar and pestle. Total RNA was isolated from the leaf powder using the RNeasy total RNA purification system from Qiagen according to the manufacturer's protocol supplied with the purification system. Poly A⁺mRNA was obtained from the total RNA obtained using an oligotex mRNA Kit purchased from Qiagen (Qiagen AG, 4052 Basel, Switzerland).

### B. Generation of a HPO lyase probe

A number of degenerate PCR primers were designed based on the amino acid sequences obtained from the HPO lyase peptides. Different primer pairs were used to amplify part of the banana HPO lyase gene. A specific amplification product was obtained, using the following sense and antisense primer pair: (multiple nucleotides at a single position reflect the degeneracy, equal amount of each nucleotide were incorporated into that position, I designates Inosine)

First strand cDNA synthesis was carried on about 100 ng of total RNA using SuperScript RNase H- Reverse Transkriptase (Gibco BRL). PCR with AmpliTaq (Perkin-Elmer) on the cDNA was performed for 40 cycles (initial heating 94°C, 3 min, annealing 50°C, 1 min, extension 72°C, 1.5 min, denaturation 94° 0.5 min; GeneAmp PCR System 2000, Perkin Elmer). The approximately 200 bp PCR product was isolated from an agarose gel and cloned into the pCR-Script SK(+) vector (Stratagene). The DNA sequence of the gene fragment was determined (commercial service: Microsynth GmbH, Balgach, Switzerland). The amino acid sequence encoded by the gene fragment is as follows:

This gene fragment was used to generate a radioactive probe. For this, 50 ng of gel-purified fragment were labeled using the BioPrime DNA labeling System (GibcoBRL) essentially as described by the manufacturer. Instead of using biotinylated nucleotides, [α32P] dCTP (50 µCi, 6000 Ci/mmol; Amersham) was used. Nonicorporated nucleotides were removed using the QIAquick Spin PCR purification kit (Qiagen).

### C. Construction of a banana leaf cDNA library

3-5 µg of banana plant mRNA were used to prepare cDNA which was then ligated into λZAP Express™ vector using the ZAP Express™ cDNA Gigapack II Gold cloning kit (Stratagene GmbH, Heidelberg, Germany). The phages obtained were then amplified before initial screening of the gene bank.

### D. Screening of the banana leaf cDNA library

About 6 x 10⁵ plaques were screened using the radioactive HPO lyase gene fragment (see above). Hybridization was done using the Quickhybe solution from Stratagene and 100 µg/ml salmon sperm DNA for 3 hrs at 68°C. Two rounds of screening were carried out. A total of 18 positive clones were obtained. The lambda vector containing the positive inserts were converted into phagemids using ExAssist Helper Phages as described (ZAP-cDNA® II Gold cloning kit, Stratagene) and plasmid DNA was isolated from all clones using the Plasmid Midi Kit (Qiagen) according to the manufacturers description.

### E. DNA sequence determination of the HPO lyase gene from banana

The DNA sequence of the phagemid insert was determined at a commercial sequencing center (MediGene GmbH, Martinsried, Germany) and is given as SEQ ID No:1.

### Example 3

### Expression of HPO lyase protein in yeast

### A. Subcloning of the cDNA into the yeast expression vector pYX233

The cDNA insert of the phagemid obtained as described above was subcloned into yeast expression vector pYX233 (R&D systems, Abingdon, UK). For this purpose oligonucleotides with one part corresponding to the C-, respectively N-terminal sequence of the cDNA and the second part harboring an appropriate restriction site recognition sequence were used. The two following oligonucleotides were synthetized (Microsynth GmbH, Balgach, Switzerland):

Using these 2 primers a PCR reaction was carried out on 10 ng Phagemid using AmpliTAQ (Perkin-Elmer) for 25 cycles with conditions as given in example 2. The 1.6 kb PCR-product was digested with the restriction enzymes Ncol and Sacl (New England Biolabs Inc.). The double digested PCR product was then purified and isolated from agarose gels using the QiaEx Kit (Qiagen). In parallel, the yeast expression vector pYX233 was linearized by digestion with Ncol and Sacl. The open vector and the purified PCR product were ligated in a 1:1 molar amount according to standard protocoll as reported by Sambrook et al., supra. The plasmid now containing the cDNA was transformed into E. coli DH5α (GibcoBRL) from which the plasmid DNA was isolated using Qiagen Plasmid Midi Kit (Qiagen, Germany).

### B. Transformation of yeast strain

5 µg of the plasmid was transformed into S. cerevisiae DBY746 (ATCC 44773) as described by Klebe et al., supra. The transformed yeast cells were plated onto suitable selective media (SD medium supplemented with the amino acids histidine, leucine, uracil; see Sherman in "Guide to yeast genetics and molecular biology", Guthrie and Fink, eds., Methods in Enzymology, Academic Press, Inc., Vol. 194, 3-21 (1991) for description of the medium) and grown for 4 days at 30°C. These cells were the source for the heterologous lyase protein. Colonies grown on the selective agar media were grown in liquid SD medium supplemented with the above amino acids. Induction of expression of the gene encoding the lyase protein was achieved by addition of 2% (final concentration) of galactose to the growth medium when culture densities had reached an absorption of 0.4 measured at 600 nm. The induction protocoll was performed essentially as described by Mylin et al. in "Gene expression technology", Goeddel, ed., Methods in Enzymology, Academic Press, Inc., Vol. 185, 297-308 (1991). Culture samples were removed 4 hr after addition of galactose and the activity of the lyase protein was measured from broken cells as described before.

### Example 4

### Production of cis-3-hexenol

S. cerevisiae cells containing the recombinant plasmid, vector pYX233 containing the HPO lyase gene as described in example 3, were cultured in 100 ml SD medium supplemented with the amino acids histidine, leucine, uracil (see Sherman, supra, for description of the medium) at 30°C. Induction conditions were as described in example 3.
The cells were harvested by centrifugation (8000 x g for 10 min) and the cell pellet was resuspended in 10 ml of 10 mM phosphate buffer, pH 6.8, 0.05% Triton-X100, 0.25 mM PMSF, 1 mM linolenic acid hydroperoxide. To the cell suspension, 10 g glass beads (0.2-0.4 mm in diameter; Sigma) were added, and the mixture was vigorously vortexed 3 times for 1 min. The reaction mixture was incubated for 30 min at room temperature, after which 0.2 g bakers yeast cells (Hefe Schweiz AG) were added. The incubation was carried out for an additional 30 min.
The reaction mixture was extracted with 10 ml of methyl-t-butylether and the organic phase separated by centrifugation (8000 x g for 10 min). The supernatant containing the produced cis-3-hexenol was saved. Cis-3-hexenol concentration was determined by capillary gas chromatography as described by Olias et al. (1993) J. Agric. Food Chem. 41, 2368-2373.

### Example 5

### Production of cis-3-hexenol and cis-3-hexenal using whole yeast cells

The lyase gene was cloned into the yeast expression vector pYX212 (R&D Systems). For this, the double digested and purified PCR product (as described in Example 3) was ligated into the vector pYX212 which was linearized with the restriction enzymes Ncol and Sacl. The plasmid which contained the PCR product was transformed into E. coli DH5α (Gibco BRL; Sambrook et al., supra) from which the plasmid DNA was isolated using Qiagen Plasmid Midi Kit (Qiagen, Germany). 5 µg of the plasmid was transformed into S. cerevisiae DBY746 (ATCC 44773) as described by Klebe et al., supra. The transformed yeast cells were plated onto suitable selective media (SD medium supplemented with the amino acids histidine, leucine, tryptophane; see Sherman, supra, for description of the medium) and grown for 4 days at 30°C. Colonies grown on the selective agar media were regrown in liquid SD medium supplemented with the above amino acids.

### Production of cis-3-hexenal

S. cerevisiae cells containing the recombinant plasmid, vector pYX212 containing the HPO lyase gene as described above, were cultured in 100 ml SD medium supplemented with the above amino acids at 30°C until culture densities reached an absorption of about 10 measured at 600 nm. The HPO lyase was expressed continuously from the constitutive triosephosphate isomerase promoter from the vector pYX212. The cells were harvested by centrifugation (8000 x g for 10 min) and resuspended in 20 ml 10 mM phosphate buffer, pH 6.8, 10 mM linolenic acid hydroperoxide. The reaction mixture was incubated for 30 min at room temperature and subsequently extracted with 10 ml of methyl-t-butylether. The organic phase was separated by centrifugation (8000 x g for 10 min) and the supernatant containing the produced cis-3-hexenal was saved.

### Production of cis-3-hexenol

S. cerevisiae cells containing the recombinant plasmid, vector pYX212 containing the HPO lyase gene as described above, were cultured in 100 ml SD medium supplemented with the above amino acids at 30°C until culture densities reached an absorption of about 10 measured at 600 nm. The HPO lyase was expressed continuously from the constitutive triosephosphate isomerase promoter from the vector pYX212. The cells were harvested by centrifugation (8000 x g for 10 min) and resuspended in 20 ml 10 mM phosphate buffer, pH 6.8, 10 mM linolenic acid hydroperoxide. 2 ml of ethanol and 10 g of commercial bakers yeast cells (Hefe Schweiz AG) were added to the resuspended recombinant yeast cells. The reaction mixture was incubated for 30 min at room temperature and subsequently extracted with 10 ml of methyl-t-butylether. The organic phase was separated by centrifugation (8000 x g for 10 min) and the supernatant containing the produced cis-3-hexenol was saved. Cis-3-hexenal and cis-3-hexenol concentrations were determined as described in Example 4.

## Claims

1. Isolated DNA sequences comprising the nucleotide sequence SEQ ID No:1 or a fragment thereof or a nucleotide sequence substantially homologous to the nucleotide sequence SEQ ID No: 1 or a fragment thereof, encoding proteins with hydroperoxide lyase activity.

2. A vector comprising a DNA sequence as claimed in claim 1.

3. A vector as claimed in claim 2 capable of directing expression in host cells selected from prokaryotic, yeast, plant and insect host cells.

4. A host transformed with a vector as claimed in claims 2 and 3 selected from the group consisting of a prokaryote, a yeast, a plant and an insect cell.

5. The host of claim 4 which is a yeast.

6. Recombinant proteins with HPO lyase activity encoded by a DNA sequence as claimed in claim 1.

7. A recombinant protein according to claim 6 comprising either the amino acid sequence SEQ ID No: 2 or an amino acid sequence substantially homologous to the amino acid sequence SEQ ID No:2.

8. A method for producing a protein as claimed in claims 6 and 7 comprising cultivating a host as claimed in claims 4 and 5 in a suitable medium and isolating said protein.

9. Recombinant proteins with HPO lyase activity whenever prepared by a process as claimed in claim 8.

10. The use of a recombinant protein according to claims 6 and 7 in the production of natural green note compounds.

11. A process for the production of green note compounds, which process comprises the steps of:
(a) reacting fatty acid hydroperoxide with recombinant proteins according to claims 6 and 7; and
(b) reacting the resulting aliphatic aldehydes with isomerases and/or alcohol dehydrogenase.

12. A process for producing cis-3-hexenol, which process comprises the steps of
(a) reacting 13-(S)-hydroperoxide linolenic acid with recombinant proteins according to claims 6 and 7; and
(b) reducing the resulting cis-3-hexenal with alcohol dehydrogenase.

13. Method for providing odorant or flavorant compositions comprising the steps of
(a) reacting fatty acid hydroperoxide with recombinant proteins according to claims 6 and 7; and
(b) reacting the resulting aliphatic aldehydes with isomerases and/or alcohol dehydrogenase; and
(c) recovering the reaction products and incorporating them into odorant or flavorant compositions in an amount sufficient to impart a green note.

14. Method for providing odorant or flavorant compositions comprising the steps of
(a) reacting 13-(S)-hydroperoxide linolenic acid with recombinant proteins according to claims 6 and 7; and
(b) reducing the resulting cis-3-hexenal with alcohol dehydrogenase; and
(c) recovering cis-3-hexenol and incorporating it into odorant or flavorant compositions in an amount sufficient to impart a green note.

## Patentansprüche

1. Isolierte DNS-Sequenzen, umfassend die Nukleotidsequenz SEQ ID No:1 oder ein Bruchstück davon oder eine Nukleotidsequenz, die im Wesentlichen zu der Nukleotidsequenz SEQ ID No:1 oder einem Fragment davon homolog ist, die Proteine mit Hydroperoxidlyaseaktivität codieren.

2. Vektor, umfassend eine DNS-Sequenz wie sie in Anspruch 1 definiert ist.

3. Vektor gemäß Anspruch 2, der zur Ausführung der Expression in Wirtszellen, ausgewählt aus prokaryotischen, Hefe-, Pflanzen- und lnsekten-Wirtszellen, eingerichtet ist.

4. Wirt, der mit einem Vektor, wie er in den Ansprüchen 2 und 3 definiert ist, transformiert ist, ausgewählt aus der Gruppe, bestehend aus einer prokaryotischen, einer Hefe-, einer Pflanzen- und einer Insektenzelle.

5. Der Wirt gemäß Anspruch 4, der eine Hefe ist.

6. Rekombinante Proteine mit HPO-Lyaseaktivität, die codiert werden durch eine DNS-Sequenz, wie sie in Anspruch 1 definiert ist.

7. Ein rekombinantes Protein gemäß Anspruch 6, umfassend entweder die Aminosäuresequenz SEQ ID No:2 oder eine Aminosäuresequenz, die im Wesentlichen zu der Aminosäuresequenz SEQ ID No:2 homolog ist.

8. Verfahren zur Herstellung eines Proteins, wie es in den Ansprüchen 6 und 7 definiert ist, umfassend ein Kultivieren eines Wirts, wie in den Ansprüchen 4 und 5 definiert, in einem geeigneten Medium und ein Isolieren des Proteins.

9. Rekombinante Proteine mit HPO-Lyaseaktivität, wenn sie nach einem Verfahren, wie in Anspruch 8 definiert, hergestellt sind.

10. Verwendung eines rekombinanten Proteins gemäß den Ansprüchen 6 und 7 bei der Herstellung von Verbindungen mit einer natürlichen Grünnote.

11. Verfahren zur Herstellung von Verbindungen mit Grünnote, wobei das Verfahren die folgenden Schritte umfasst:
(a) Umsetzen eines Fettsäurehydroperoxids mit rekombinanten Proteinen gemäß den Ansprüchen 6 und 7; und
(b) Umsetzen der erhaltenen aliphatischen Aldehyde mit lsomerasen und/oder Alkoholdehydrogenase.

12. Verfahren zur Herstellung von cis-3-Hexenol, wobei das Verfahren die folgenden Schritte umfasst:
(a) Umsetzen von 13-(S)-Hydroperoxidlinolensäure mit rekombinanten Proteinen gemäß den Ansprüchen 6 und 7; und
(b) Reduzieren des erhaltenen cis-3-Hexenals mit Alkoholdehydrogenase.

13. Verfahren zur Bereitstellung von Odorisierungsmittel- oder Geschmacksmittelzusammensetzungen, umfassend die folgenden Schritte:
(a) Umsetzen von Fettsäurehydroperoxid mit rekombinanten Proteinen gemäß den Ansprüchen 6 und 7; und
(b) Umsetzen der erhaltenen aliphatischen Aldehyde mit Isomerasen und/oder Alkoholdehydrogenase; und
(c) Gewinnung der Reaktions- oder Umsetzungsprodukte und Einverleibung derselben in Odorisierungsmittel- oder Geschmacksmittelzusammensetzungen in einer Menge, die ausreicht, um eine Grünnote zu verleihen.

14. Verfahren zur Bereitstellung von Odorisierungsmittel- oder Geschmacksmittelzusammensetzungen, umfassend die Schritte:
(a) Umsetzen von 13-(S)-Hydroperoxidlinolensäure mit rekombinanten Proteinen gemäß den Ansprüchen 6 und 7; und
(b) Reduzieren des erhaltenen cis-3-Hexenals mit Alkoholdehydrogenase; und
(c) Gewinnen von cis-3-Hexenol und Einverleiben desselben in Odorisierungsmittel- oder Geschmacksmittelzusammensetzungen in einer Menge, die ausreicht, um eine Grünnote zu verleihen.

## Revendications

1. Séquences d'ADN isolées comprenant la séquence nucléotidique SEQ ID N°1 ou un de ses fragments ou une séquence nucléotidique substantiellement homologue à la séquence nucléotidique SEQ ID N° 1 ou un de ses fragments, codant pour des protéines ayant une activité d'hydroperoxyde-lyase.

2. Vecteur comprenant une séquence d'ADN telle que revendiquée dans la revendication 1.

3. Vecteur tel que revendiqué dans la revendication 2, capable de diriger l'expression dans des cellules hôtes choisies parmi les cellules hôtes procaryotes, de levure, de plante et d'insecte.

4. Hôte transformé avec un vecteur tel que revendiqué dans les revendications 2 et 3, choisi dans le groupe constitué par un procaryote, une levure, une plante et une cellule d'insecte.

5. Hôte selon la revendication 4 qui est une levure.

6. Protéines recombinantes ayant une activité de HPO-lyase, codées par une séquence d'ADN telle que revendiquée dans la revendication 1.

7. Protéine recombinante selon la revendication 6, comprenant soit la séquence d'acides aminés SEQ ID N°2 soit une séquence d'acides aminés substantiellement homologue à la séquence d'acides aminés SEQ ID N°2.

8. Procédé de production d'une protéine telle que revendiquée dans les revendications 6 et 7, comprenant la mise en culture d'un hôte tel que revendiqué dans les revendications 4 et 5 dans un milieu approprié et l'isolement de ladite protéine.

9. Protéines recombinantes ayant une activité HPO-lyase quand elles sont préparées par un procédé tel que revendiqué dans la revendications 8.

10. Utilisation d'une protéine recombinante selon les revendications 6 et 7 dans la production de composés d'une note verte naturelle.

11. Procédé pour la production de composés d'une note verte, lequel procédé comprend les étapes consistant à:
(a) faire réagir de l'hydroperoxyde d'acide gras avec des protéines recombinantes selon les revendications 6 et 7; et
(b) faire réagir les aldéhydes aliphatiques résultants avec des isomérases et/ou une alcool-déshydrogénase.

12. Procédé de production de cis-3-hexénol, lequel procédé comprend les étapes consistant à:
(a) faire réagir l'acide 13-(S)-hydroperoxyde-linolénique avec des protéines recombinantes selon les revendications 6 et 7; et
(b) réduire le cis-3-hexénal résultant avec de l'alcool-déshydrogénase.

13. Procédé d'obtention de compositions odorantes ou aromatisantes, comprenant les étapes consistant à:
(a) faire réagir de l'hydroperoxyde d'acide gras avec des protéines recombinantes selon les revendications 6 et 7; et
(b) faire réagir les aldéhydes aliphatiques résultants avec des isomérases et/ou une alcool-déshydrogénase; et
(c) récupérer les produits réactionnels et les incorporer dans des compositions odorantes ou aromatisantes en une quantité suffisante pour conférer une note verte.

14. Procédé d'obtention de compositions odorantes ou aromatisantes, comprenant les étapes consistant à:
(a) faire réagir l'acide 13-(S)-hydroperoxyde-linolénique avec des protéines recombinantes selon les revendications 6 et 7; et
(b) réduire le cis-3-hexénal résultant avec de l'alcool-déshydrogénase, et
(c) récupérer le cis-3-hexénol et l'incorporer dans des compositions odorantes ou aromatisantes en une quantité suffisante pour conférer une note verte.


### SEQUENCE LISTING

(1) GENERAL INFORMATION:
   (i) APPLICANT:
      (A) NAME: GIVAUDAN-ROURE (INTERNATIONAL) SA
      (B) STREET: Ch.Parfumerie 5
      (C) CITY: Vernier
      (D) STATE: Geneva
      (E) COUNTRY: Switzerland
      (F) POSTAL CODE (ZIP): CH-1214
      (G) TELEPHONE: 061-688 42 56
      (H) TELEFAX: 061-688 13 95
      (I) TELEX: 962292/965542 hlr ch
   (ii) TITLE OF INVENTION: Hydroperoxide Lyases
   (iii) NUMBER OF SEQUENCES: 2
   (iv) COMPUTER READABLE FORM:
      (A) MEDIUM TYPE: Floppy disk
      (B) COMPUTER: Apple Macintosh
      (C) OPERATING SYSTEM: System 7.1 (Macintosh)
      (D) SOFTWARE: World 5.0
(2) INFORMATION FOR SEQ ID NO: 1:
   (A) LENGTH: 1638 base pairs
   (B) TYPE: nucleic acid
   (C) STRANDEDNESS: single
   (D) TOPOLOGY: linear

   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:25..1473
   (xi) SEQUENCE DESCRIPTION: SEQ ID NO: 1:
   AAGAAGAAGA GAGGGAAGGT ACGG ATG GCT ATG ATG TGG TCG TCA GCC TCC          51
                              Met Ala Met Met Trp Ser Ser Ala Ser
                                1               5
   GCC ACC GCC GTC ACC ACG CTG CCG ACG AGG CCC ATC CCT GGA AGC TAC          99
   Ala Thr Ala Val Thr Thr Leu Pro Thr Arg Pro Ile Pro Gly Ser Tyr
    10                  15                  20                  25
   GGC CCG CCG CTG GTG GGC CCC CTC AAG GAC CGC CTC GAC TAC TTC TGG        147
   Gly Pro Pro Leu Val Gly Pro Leu Lys Asp Arg Leu Asp Tyr Phe Trp
                   30                  35                  40
   TTT CAG GGA CCG GAG ACC TTC TTC CGC AGC CGG ATG GCC ACC CAC AAG        195
   Phe Gln Gly Pro Glu Thr Phe Phe Arg Ser Arg Met Ala Thr His Lys
               45                  50                  55
   AGC ACC GTG TTC CGC ACC AAC ATG CCC CCC ACC TTC CCC TTC TTC GTT        243
   Ser Thr Val Phe Arg Thr Asn Met Pro Pro Thr Phe Pro Phe Phe Val
           60                  65                  70
   GGA GTC GAC CCC CGC GTG GTC ACC GTC CTC GAC TGC ACA TCC TTC TCC        291
   Gly Val Asp Pro Arg Val Val Thr Val Leu Asp Cys Thr Ser Phe Ser
       75                  80                  85
   GCC CTC TTC GAC CTC GAG GTC GTG GAG AAG AAG AAC ATT CTC ATC GGG        339
   Ala Leu Phe Asp Leu Glu Val Val Glu Lys Lys Asn Ile Leu Ile Gly
    90                  95                 100                 105
   GAC TAC ATG CCC AGC CTC AGC TTC ACC GGC GAC ACC CGC GTC GTC GTG        387
   Asp Tyr Met Pro Ser Leu Ser Phe Thr Gly Asp Thr Arg Val Val Val
                   110                 115                 120
   TAC CTC GAC CCC TCC GAG CCC GAC CAC GCC CGC GTG AAG AGC TTC TGC        435
   Tyr Leu Asp Pro Ser Glu Pro Asp His Ala Arg Val Lys Ser Phe Cys
               125                 130                 135
   TTG GAA CTC CTC AGG CGC GGC GCC AAG ACC TGG GTC TCC TCG TTC CTC        483
   Leu Glu Leu Leu Arg Arg Gly Ala Lys Thr Trp Val Ser Ser Phe Leu
           140                 145                 150
   TCC AAT CTC GAT GTC ATG CTC GCC ACC ATA GAG CAG GGG ATC GCC AAG        531
   Ser Asn Leu Asp Val Met Leu Ala Thr Ile Glu Gln Gly Ile Ala Lys
       155                 160                 165
   GAT GGC TCC GCC GGC TTA TTC GGC CCG CTG CAG AAG TGC ATC TTC GCG        579
   Asp Gly Ser Ala Gly Leu Phe Gly Pro Leu Gln Lys Cys Ile Phe Ala
   170                 175                 180                 185
   TTC CTC TGC AAG AGC ATC ATC GGG GCC GAC CCG TCG GTG TCG CCC GAC        627
   Phe Leu Cys Lys Ser Ile Ile Gly Ala Asp Pro Ser Val Ser Pro Asp
                   190                 195                 200
   GTG GGA GAA AAT GGC TTC GTC ATG CTC GAC AAG TGG CTT GCG CTG CAG        675
   Val Gly Glu Asn Gly Phe Val Met Leu Asp Lys Trp Leu Ala Leu Gln
               205                 210                 215
   CTC CTC CCG ACG GTG AAG GTC GGG GCC ATC CCG CAA CCC CTG GAG GAG        723
   Leu Leu Pro Thr Val Lys Val Gly Ala Ile Pro Gln Pro Leu Glu Glu
           220                 225                 230
   ATC CTC CTC CAC TCC TTC CCC CTC CCC TTC TTC CTC GTG AGC CGC GAT        771
   Ile Leu Leu His Ser Phe Pro Leu Pro Phe Phe Leu Val Ser Arg Asp
       235                 240                 245
   TAC CGG AAG CTG TAC GAA TTC GTC GAG AAG CAA GGC CAA GAG GTT GTC        819
   Tyr Arg Lys Leu Tyr Glu Phe Val Glu Lys Gln Gly Gln Glu Val Val
   250                 255                 260                 265
   CGG CGA GCG GAA ACC GAG CAC GGG CTC AGC AAG CAC GAC GCC ATC AAC      867
   Arg Arg Ala Glu Thr Glu His Gly Leu Ser Lys His Asp Ala Ile Asn
                   270                 275                 280
   AAC ATC TTG TTC GTC CTA GGA TTC AAC GCC TTC GGC GGC TTC TCG GTC      915
   Asn Ile Leu Phe Val Leu Gly Phe Asn Ala Phe Gly Gly Phe Ser Val
               285                 290                 295
   TTC TTC CCC ACG CTC CTG ACC ACC ATA GGG AGG GAC AAG ACG GGC CTG      963
   Phe Phe Pro Thr Leu Leu Thr Thr Ile Gly Arg Asp Lys Thr Gly Leu
           300                 305                 310
   CGG GAG AAG CTC AAG GAC GAG GTG CGC AGG GTC ATG AAG AGT AGA GGG     1011
   Arg Glu Lys Leu Lys Asp Glu Val Arg Arg Val Met Lys Ser Arg Gly
       315                 320                 325
   GAG AAG CGG CCG AGC TTC GAG ACG GTG CGG GAG ATG GAG CTG GTG CGA     1059
   Glu Lys Arg Pro Ser Phe Glu Thr Val Arg Glu Met Glu Leu Val Arg
   330                 335                 340                 345
   TCG ACG GTG TAC GAG GTC CTG CGG CTG AAC CCG CCG GTG CCG CTG CAG     1107
   Ser Thr Val Tyr Glu Val Leu Arg Leu Asn Pro Pro Val Pro Leu Gln
                   350                 355                 360
   TAC GGG CGG GCG CGC ACC GAC TTC ACG CTG AAC TCC CAC GAC GCG GCG     1155
   Tyr Gly Arg Ala Arg Thr Asp Phe Thr Leu Asn Ser His Asp Ala Ala
               365                 370                 375
   TTC AAG GTT GAG AAG GGG GAG TTG CTG TGC GGG TAC CAG CCG CTG GTG     1203
   Phe Lys Val Glu Lys Gly Glu Leu Leu Cys Gly Tyr Gln Pro Leu Val
           380                 385                 390
   ATG CGG GAT CCA GCG GTG TTC GAC GAC CCG GAG ACG TTC GCC CCG GAA     1251
   Met Arg Asp Pro Ala Val Phe Asp Asp Pro Glu Thr Phe Ala Pro Glu
       395                 400                 405
   AGG TTC ATG GGC AGC GGG AAG GAG CTG CTC AAG TAC GTC TTC TGG TCC     1299
   Arg Phe Met Gly Ser Gly Lys Glu Leu Leu Lys Tyr Val Phe Trp Ser
   410                 415                 420                 425
   AAC GGG CCG GAG ACG GGT ACG CCG ACG CCG GCC AAC AAG CAG TGC GCC     1347
   Asn Gly Pro Glu Thr Gly Thr Pro Thr Pro Ala Asn Lys Gln Cys Ala
                   430                 435                 440
   GCG AAG GAC TAC GTG GTG GAG ACG GCG TGC CTG CTG ATG GCG GAG ATC     1395
   Ala Lys Asp Tyr Val Val Glu Thr Ala Cys Leu Leu Met Ala Glu Ile
               445                 450                 455
   TTC TAC CGC TAC GAC GAG TTC GTG TGC GCC GAC GAC GCC ATC TCC GTG     1443
   Phe Tyr Arg Tyr Asp Glu Phe Val Cys Ala Asp Asp Ala Ile Ser Val
           460                 465                 470
   ACG AAG CTG GAT AGA GCG AGA GAA TGG GAG TAA ACGGTATTCA AGTCGGAAGC   1496
   Thr Lys Leu Asp Arg Ala Arg Glu Trp Glu  *
       475                 480
   GACATAAGGA GACGGCCAAC TCCACCGTTG CTAATTCAAG TCGTACTCCA AATCGGTATT   1556
   CATATCATCG TTCCATTGGG GTGATGAAGA GATAAATAAA ATTTGACGTT GCAGGAGGCT   1616
   ACAAAAAAAA AAAAAAAAAA AA                                            1638
(2) INFORMATION FOR SEQ ID NO: 2:
   (i) SEQUENCE CHARACTERISTICS:
      (A) LENGTH: 483 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ala Met Met Trp Ser Ser Ala Ser Ala Thr Ala Val Thr Thr Leu
    1               5                  10                  15
   Pro Thr Arg Pro Ile Pro Gly Ser Tyr Gly Pro Pro Leu Val Gly Pro
               20                  25                  30
   Leu Lys Asp Arg Leu Asp Tyr Phe Trp Phe Gln Gly Pro Glu Thr Phe
           35                  40                  45
   Phe Arg Ser Arg Met Ala Thr His Lys Ser Thr Val Phe Arg Thr Asn
       50                  55                  60
   Met Pro Pro Thr Phe Pro Phe Phe Val Gly Val Asp Pro Arg Val Val
   65                  70                  75                  80
   Thr Val Leu Asp Cys Thr Ser Phe Ser Ala Leu Phe Asp Leu Glu Val
                   85                  90                  95
   Val Glu Lys Lys Asn Ile Leu Ile Gly Asp Tyr Met Pro Ser Leu Ser
               100                 105                 110
   Phe Thr Gly Asp Thr Arg Val Val Val Tyr Leu Asp Pro Ser Glu Pro
           115                 120                 125
   Asp His Ala Arg Val Lys Ser Phe Cys Leu Glu Leu Leu Arg Arg Gly
       130                 135                 140
   Ala Lys Thr Trp Val Ser Ser Phe Leu Ser Asn Leu Asp Val Met Leu
   145                 150                 155                 160
   Ala Thr Ile Glu Gln Gly Ile Ala Lys Asp Gly Ser Ala Gly Leu Phe
                   165                 170                 175
   Gly Pro Leu Gln Lys Cys Ile Phe Ala Phe Leu Cys Lys Ser Ile Ile
               180                 185                 190
   Gly Ala Asp Pro Ser Val Ser Pro Asp Val Gly Glu Asn Gly Phe Val
           195                 200                 205
   Met Leu Asp Lys Trp Leu Ala Leu Gln Leu Leu Pro Thr Val Lys Val
       210                 215                 220
   Gly Ala Ile Pro Gln Pro Leu Glu Glu Ile Leu Leu His Ser Phe Pro
   225                 230                 235                 240
   Leu Pro Phe Phe Leu Val Ser Arg Asp Tyr Arg Lys Leu Tyr Glu Phe
                   245                 250                 255
   Val Glu Lys Gln Gly Gln Glu Val Val Arg Arg Ala Glu Thr Glu His
               260                 265                 270
   Gly Leu Ser Lys His Asp Ala Ile Asn Asn Ile Leu Phe Val Leu Gly
           275                 280                 285
   Phe Asn Ala Phe Gly Gly Phe Ser Val Phe Phe Pro Thr Leu Leu Thr
       290                 295                 300
   Thr Ile Gly Arg Asp Lys Thr Gly Leu Arg Glu Lys Leu Lys Asp Glu
   305                 310                 315                 320
   Val Arg Arg Val Met Lys Ser Arg Gly Glu Lys Arg Pro Ser Phe Glu
                   325                 330                 335
   Thr Val Arg Glu Met Glu Leu Val Arg Ser Thr Val Tyr Glu Val Leu
               340                 345                 350
   Arg Leu Asn Pro Pro Val Pro Leu Gln Tyr Gly Arg Ala Arg Thr Asp
           355                 360                 365
   Phe Thr Leu Asn Ser His Asp Ala Ala Phe Lys Val Glu Lys Gly Glu
       370                 375                 380
   Leu Leu Cys Gly Tyr Gln Pro Leu Val Met Arg Asp Pro Ala Val Phe
   385                 390                 395                 400
   Asp Asp Pro Glu Thr Phe Ala Pro Glu Arg Phe Met Gly Ser Gly Lys
                   405                 410                 415
   Glu Leu Leu Lys Tyr Val Phe Trp Ser Asn Gly Pro Glu Thr Gly Thr
               420                 425                 430
   Pro Thr Pro Ala Asn Lys Gln Cys Ala Ala Lys Asp Tyr Val Val Glu
           435                 440                 445
   Thr Ala Cys Leu Leu Met Ala Glu Ile Phe Tyr Arg Tyr Asp Glu Phe
       450                 455                 460
   Val Cys Ala Asp Asp Ala Ile Ser Val Thr Lys Leu Asp Arg Ala Arg
   465                 470                 475                 480
   Glu Trp Glu  *